# EUROPÄISCHE PATENTSCHRIFT

(11) **EP 3 833 425 B1**
(45) Veröffentlichungstag und Bekanntmachung des Hinweises auf die Patenterteilung: **08.06.2022**
(21) Anmeldenummer: 19758628.2
(22) Anmeldetag: 05.08.2019
(51) Int. Cl.: A61N 1/05, A61N 1/36

(54) **MEDIZINISCHES IMPLANTAT IN ART EINER WICKELMANSCHETTEN-ELEKTRODENANORDNUNG**
MEDICAL IMPLANT OF THE TYPE OF A WRAP-AROUND CUFF ELECTRODE ASSEMBLY
IMPLANT MÉDICAL SOUS FORME D'UN AGENCEMENT D'ÉLECTRODES À MANCHON D'ENROULEMENT

(30) Priorität: 06.08.2018 DE 102018213120
(43) Veröffentlichungstag der Anmeldung: 16.06.2021
(73) Patentinhaber: Neuroloop GmbH, 79110 Freiburg (DE)
(72) Erfinder: PLACHTA, Dennis, 79279 Vörstetten (DE); KIMMIG, Fabian, 79331 Teningen (DE); BORETIUS, Tim, 79098 Freiburg (DE)
(74) Vertreter: Rösler, Uwe
(86) Internationale Anmeldenummer: PCT/EP2019/071025
(87) Internationale Veröffentlichungsnummer: WO 2020/030592

(56) Entgegenhaltungen:
- WO-A1-2013/159136
- WO-A1-2016/055512
- CA-A1- 2 139 097
- DE-A1-102016 222 712
- US-A- 5 938 596
- US-A1- 2012 192 874
- US-A1- 2012 277 819

## Beschreibung

### Technisches Gebiet

Die Erfindung bezieht sich auf ein medizinisches Implantat in Art einer Wickelmanschetten-Elektrodenanordnung, kurz Cuff-Elektrode, die ein flexibles, biokompatibles, folienartiges Trägersubstrat aufweist, das ersten Trägersubstratbereich aufweist, der durch Wickeln um eine Wickelachse die Gestalt eines Tubus annimmt.

### Stand der Technik

Elektronische Implantate, die für einen dauerhaften oder zumindest langfristigen Verbleib im Körper geeignet sind, werden typischerweise zur therapeutischen Beeinflussung von Organfunktionen eingesetzt. Unter der großen Vielzahl unterschiedlich ausgebildeter und für jeweils unterschiedliche therapeutische Anwendungsziele speziell konfektionierter Implantate beziehen sich die weiteren Betrachtungen vornehmlich auf ein als eine Wickelmanschetten-Elektrodenanordnung ausgebildetes Implantat, auch als Cuff-Elektrode bezeichnet, das speziell für eine intrakorporale Anbringung und möglichst dauerhafte Befestigung längs eines Nervenfaserbündels ausgebildet ist, um zumindest elektrische Stimulationssignale am Nervenfaserbündel bedarfsgerecht zu applizieren.

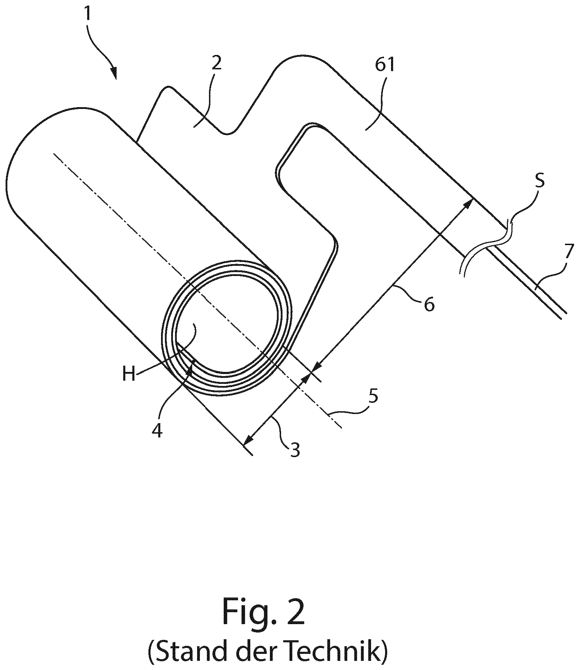

Ein gattungsgemäßes medizinisches Implantat, das in Art einer Wickelmanschette zum Zwecke der Detektion sowie auch Applikation neuronaler elektrischer Signale für einen dauerhaften oder zumindest langfristigen Verbleib längs eines Nervenfaserbündels innerhalb des menschlichen oder tierischen Körpers geeignet ausgebildet ist, ist in der Druckschrift WO 2016/055512 A1 beschrieben sowie in Figur 2 schematisiert dargestellt. Das aus einem biokompatiblen, flächigen bzw. folienartigen Trägersubstrat 2 gefertigte und als Wickelmanschette ausgebildete medizinische Implantat 1 weist einen ersten Trägersubstratbereich 3 auf, der um eine Raumachse 5 unter Ausbildung wenigstens einer Substratwicklung, vorzugsweise unter Ausbildung eineinhalb-, zwei- oder mehrfacher Substratwicklungen, gewickelt ist, die einen geradzylinderförmigen Hohlraum H radial umfasst. An der dem geradzylindrigen Hohlraum H zugewandten Substratoberfläche ist eine Vielzahl nicht in Figur 2 dargestellter Elektrodenflächen angebracht, die in mittel- oder unmittelbaren körperlichem Kontakt mit dem Epineurium eines von der Wickelmanschette 1 umfassten Nervenfaserbündels tritt, ebenfalls in Figur 2 nicht dargestellt. Um zu gewährleisten, dass das medizinische Implantat 1 einerseits möglichst ortsfest längs eines Nervenfaserbündels nach entsprechender Implantation verbleibt und andererseits den natürlichen Formänderungen eines Nervenfaserbündels zu folgen vermag oder diesem zumindest keinen nennenswerten mechanischen Widerstand entgegensetzt, liegen die einzelnen Substratwicklungen lose aneinander an und vermögen im Falle einer Ausdehnung des Nervenfaserbündels durch entsprechende Relativbewegung den Durchmesser des umfassten Hohlraumes H zu vergrößern.

Durch eine dem Trägersubstrat 2 im einem ersten Trägersubstratbereich 3 eingeprägte Materialvorspannung nimmt das Trägersubstrat 2 ohne äußere Krafteinwirkung eine vordefinierte Wickelkonfiguration ein, bei der das freie Abschnittsende 4 wenigstens einlagig lose von dem ersten Trägersubstratbereich 3, d.h. von wenigstens einer Substratwicklung radial überdeckt ist.

An dem gewickelten ersten Trägersubstratbereich 3 schließt in dieser Ausführungsform einstückig ein nicht um die Raumachse 5 gewickelter zweiter Trägersubstratbereich 6 an, innerhalb dem elektrische Zu- bzw. Ableitungen geführt sind, die zu der Vielzahl der mit dem Epineurium des Nervenfaserbündels mittel- oder unmittelbar in Kontakt tretenden Elektroden verbunden sind. Der gleichfalls flächig ausgebildete zweite Trägersubstratbereich 6 weist im dargestellten Ausführungsbeispiel einen parallel zur Raumachse 5 orientierten stegförmig ausgebildeten Flächenabschnitt 61 auf, der über eine nicht weiter dargestellte Schnittstelle S, beispielsweise in Form einer implantierbaren Steckerverbindung S, mit einer von dem medizinischen Implantat 1 wegführenden Verbindungsstruktur 7 verbunden ist, längs der die elektrischen Leitungen zu einer separaten, vorzugsweise implantierbaren Versorgungseinheit in Form eines Steuergerätes bzw. einer Energiequelle geführt werden.

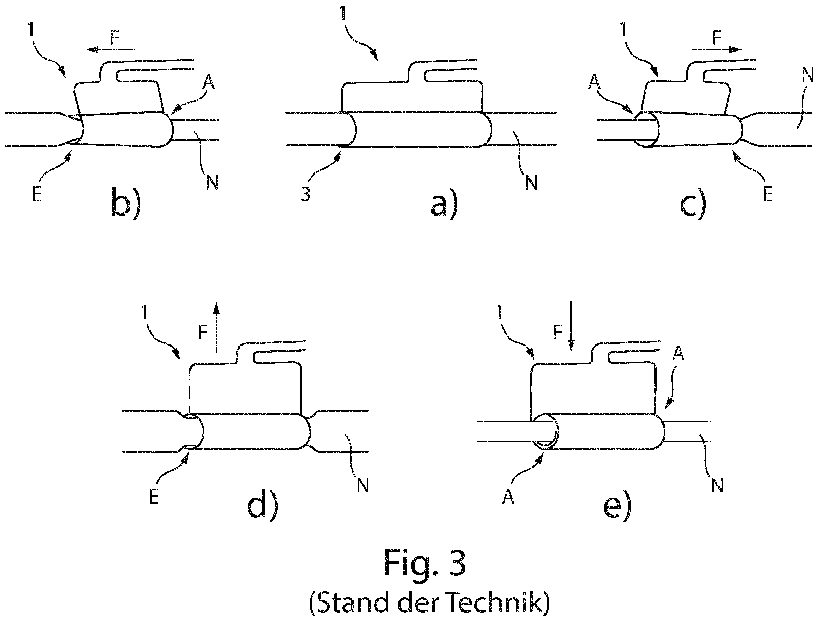

In einem unbelasteten Zustand sitzt das medizinische Implantat 1 längs eines Nervenfaserbündels N gemäß der in Figur 3a schematisierten Darstellung mit einer im Durchmesser einheitlichen Umfassung des zu einer Wickelmanschette geformten ersten Trägersubstratabschnittes 3 an. In diesem Zustand wirkt kein oder nur ein minimaler mechanischer äußerer Zwang auf das Nervenfaserbündel N ein. Wirken hingegen externe Kräfte F auf das medizinische Implantat 1 ein, die beispielsweise von körpereigenen Bewegungen herrühren, so kann es zu Verformungen der Wickelgeometrie längs des als Wickelmanschette ausgebildeten ersten Substratabschnittes 3 kommen, wodurch zum einen die Befestigung des medizinischen Implantates längs des Nervenfaserbündels N nicht mehr sichergestellt sein kann und zum anderen ein mechanischer Stress durch das medizinische Implantat auf das Nervenfaserbündel einwirken kann. In den Figuren 3b bis e sind derartige Belastungssituationen skizziert. So führen beispielsweise Zugkräfte F, die im Wesentlichen parallel zur Längserstreckung des Nervenfaserbündels N orientiert sind, innerhalb der Wickelmanschette zu einer trichterförmigen Verformung, die einerseits zu einer Einschnürung E am Nervenfaserbündel N und andererseits zu einer Aufweitung A und damit verbundenen radialen Beabstandung der Wickelmanschette vom Nervenfaserbündel N führen, siehe hierzu Figuren 3b und c. Zu einer Einschnürung E des Nervenfaserbündels N kann es ebenfalls im Falle einer orthogonal zur Nervenfaserbündellängserstreckung orientierten Krafteinwirkung F auf das medizinische Implantat 1 kommen, siehe die in Figur 3d illustrierte Belastungssituation. In diesem Fall wirkt die Kraft F als Zugkraft quer zum Nervenfaserbündel N. In Figur 1e wirkt die Kraft F in umgekehrter Kraftrichtung, in Form einer auf das Nervenfaserbündel N gerichteten Schubkraft, wodurch die Wickelmanschette im Durchmesser aufgeweitet und vom Nervenfaserstrang N tendenziell gelöst wird.

Langfristig implantierte gattungsgemäße Cuff-Elektroden unterliegen überdies einen weiteren nachteiligen Einfluss, der zu funktionalen Beeinträchtigungen sowie zur Erschwerung einer möglicherweise erforderlichen chirurgischen Entfernung der Cuff-Elektrode vom Nervenfaserbündel führen kann und durch das natürliche Gewebewachstum bedingt ist, wodurch die Cuff-Elektrode längs des Nervenfaserbündels regelrecht einwächst bzw. von Gewebe vollständig überwuchert wird. Aufgrund der losen Trägersubstratwicklung innerhalb des zu einem Tubus gewickelten ersten Trägersubstratbereiches können so wachstumsbedingt Gewebebereiche auch zwischen einzelnen Substratwicklungslagen eindringen.

Eine andere Art von Cuff-Elektrode ist in der US 2013/0123895 A1 erläutert, die im Gegensatz zu einer Wickelelektrode einen quaderförmigen Grundkörper aufweist, an dem zwei lappenartig ausgebildete Flächenstücke angebracht sind, die einander einlagig überlappend einen Nerv umschlingen. Durch die gegenseitige Umschlingung beider Flächenstücke stabilisiert das radial außen liegende Flächenstück das radial innenliegende Flächenstück.

Die Druckschrift CA 2 139 097 A1 offenbart eine implantierbare, zylindermantelartige Cuff-Anordnung, zu deren Anbringung an einem Nervenstrang der Cuff-Zylinder einen Längsschnitt mit gegenseitig ineinandergreifenden und miteinander längsfluchtenden Befestigungshülsen besitzt, in die ein Sicherungsstift zum Verschluss des Cuff-Zylinders einbringbar ist.

### Darstellung der Erfindung

Der Erfindung liegt die Aufgabe zugrunde ein medizinisches Implantat in Art einer Wickelmanschetten-Elektrodenanordnung, kurz Cuff-Elektrode, die ein flexibles, biokompatibles, folienartiges Trägersubstrat aufweist, das einen Trägersubstratbereich aufweist, der durch Wickeln um eine Wickelachse die Gestalt eines Tubus annimmt, derart weiterzubilden, so dass die vorstehenden Nachteile bezüglich zugkraftbedingter, mechanischer Belastungen und die dadurch hervorgerufenen Wickelmanschettendeformationen, sowie das Gewebewachstum bedingte Überwuchern der Cuff-Elektrode und die damit verbundenen Verletzungsrisiken bei einer erforderlichen chirurgischen Entfernung des Implantats vom Nervenfaserstranges, signifikant reduziert bzw. vollständig vermieden werden sollen. Zudem gilt es eine Sicherung für das Implantat gegen Dislozierung und vollständiges Ablösen vom intrakorporalen Gefäß oder Nervenfaserbündel vorzusehen.

Die Lösung der der Erfindung zugrundeliegende Aufgabe ist im Anspruch 1 angegeben. Den Erfindungsgedanken in vorteilhafter Weise ausbildende Merkmale sind in den Unteransprüchen angegeben.

Lösungsgemäß zeichnet sich ein medizinisches Implantat in Art einer Cuff-Elektrode mit den Merkmalen des Oberbegriffes des Anspruches 1 dadurch aus, dass eine Schutzstruktur unmittelbar oder mittelbar an dem Trägersubstrat angebracht ist, die aus einem ersten, geöffneten Zustand in einen zweiten, geschlossenen und den zu einem Tubus gewickelten Trägersubstratbereich zumindest axial zur Wickelachse bereichsweise und in Umfangsrichtung des Tubus vollständig umfassenden Zustand überführbar ist.

Die der lösungsgemäß ausgebildeten Schutzstruktur zugrundeliegende Idee betrifft eine Umhüllung oder Ummantelung für die längs eines Nervenstranges angebrachte Cuff-Elektrode, zum Zwecke eines mechanischen Schutzes und zusätzlichen Haltens der Cuff-Elektrode, d. h. insbesondere für den zu einem gradzylinderförmigen Tubus gewickelten und den Nervenstrang unmittelbar berührenden Trägersubstratbereich. Zudem vermag die Schutzstruktur eine Barriere gegen die Cuff-Elektrode überwucherndes Zell- bzw. Gewebewachstum zu bilden.

Zum Zwecke einer für den Arzt möglichst einfachen Handhabung ist die lösungsgemäße Schutzstruktur untrennbar entweder unmittelbar an dem Trägersubstrat der Cuff-Elektrode oder unmittelbar an einer elektrischen Zu- und Ableitungsstruktur angebracht, die vorzugsweise über eine lösbar feste Fügeverbindung mit der Cuff-Elektrode verbindbar ist. Auf diese Weise kann der Arzt die mittel- oder unmittelbar am Trägersubstrat angebrachte Schutzstruktur in zeitlicher Abfolge unmittelbar nach Anlegen der Cuff-Elektrode um den Nervenstrang applizieren und dies in einer konstruktiv exakt vorgegebenen Lage und Position zur Cuff-Elektrode. Ein zeitaufwendiges Positionieren und Zentrieren der Schutzstruktur relativ zur Cuff-Elektrode entfällt aufgrund der räumlich fest vorgegebenen Zuordnung zwischen Schutzstruktur und Cuff-Elektrode. Zudem ist sichergestellt, dass eine Separation der Schutzstruktur vom Implantat ausgeschlossen ist.

Vorzugsweise ist die Schutzstruktur über wenigstens einen stegartig ausgebildeten Verbindungsabschnitt mittel- oder unmittelbar mit dem Trägersubstrat verbunden. Im Falle der unmittelbaren Anbringung der Schutzstruktur am Trägersubstrat ist die vorzugsweise folienartig ausgebildete Schutzstruktur monolithisch über den wenigstens einen stegartig ausgebildeten Verbindungsabschnitt mit dem Trägersubstrat verbunden.

Die Anbringung sowie auch die Form- und Größendimensionierung des wenigstens einen Verbindungsabschnittes ist vorzugsweise derart gewählt, so dass die folienartig ausgebildete Schutzstruktur eine eindeutige Position und Lage relativ zu dem zu einem Tubus gewickelten ersten Trägersubstratbereich einnimmt, so dass der Arzt eindeutig und sicher die Schutzstruktur aus dem ersten geöffneten Zustand zum Zwecke der Umhüllung bzw. Ummantelung in den zweiten geschlossenen und den zu einem Tubus gewickelten ersten Substratbereich umfassenden Zustand überführen kann, in dem die Schutzstruktur den zu einem Tubus gewickelten ersten Trägersubstratbereich, vorzugsweise axial und in Umfangsrichtung vollständig überdeckt. Vorzugsweise ist die axiale Erstreckung der Schutzstruktur größer als die der Cuff-Elektrode bemessen, so dass die Schutzstruktur im geschlossenen, zweiten Zustand den zu einem Tubus gewickelten zweiten Trägersubstratbereich der Cuff-Elektrode axial beidseitig jeweils mit einem Überlapp überragt. Hierdurch kann sicher vermieden werden, dass die Cuff-Elektrode im Laufe der Zeit von umliegendem Gewebe überwachsen bzw. überwuchert wird.

Je nach Ausbildung und Ausprägung des folienartigen Trägersubstrats der Cuff-Elektrode kann die Schutzstruktur auch unmittelbar, d.h. ohne Vorsehen eines stegartigen Verbindungsabschnittes, vorzugsweise monolithisch, mit dem Trägersubstrat verbunden sein.

Um sicherzustellen, dass die sich im zweiten, geschlossenen Zustand befindliche Schutzstruktur nicht unkontrolliert von der Cuff-Elektrode zu lösen vermag, sieht ein bevorzugtes Ausführungsbeispiel einen an der Schutzstruktur angebrachten Fügemechanismus vor, der unter Ausbildung einer Form- und/oder Kraftschlussverbindung die Schutzstruktur im zweiten, geschlossenen Zustand sichert. Der Fügemechanismus ist vorzugsweise in Form eines Rastmechanismus realisiert. Auch können klettartige Verbindungsstrukturen und/oder band- oder fadenartige Befestigungsmittel an der Schutzstruktur angebracht sein, die unter Schlaufen- und/oder Knotenbildung für einen dauerhaft festen Halt der Schutzstruktur an der Cuff-Elektrode sorgen.

Die Schutzstruktur sieht in einer bevorzugten Ausführungsform zwei zylinderförmige Halbschalen vor, die in gegenseitiger Ergänzung bzw. Überlappung einen geradzylinderförmigen Hohlraum einschließen, in den der zu einem Tubus gewickelte Trägersubstratbereich samt Nervenstrang, um den die Cuff-Elektrode angelegt ist, nahtlos Platz finden.

Die zwei zylinderförmigen Halbschalen sind vorzugsweise einstückig, d. h. monolithisch miteinander verbunden, vorzugsweise über ein Festkörpergelenk und aus dem geöffneten Zustand in einen den zylinderförmigen Hohlraum umfassenden, geschlossenen Zustand durch einen bloßen Klappvorgang überführbar. Im Bereich der sich unmittelbar angrenzenden oder teilweise überlappenden Halbschalenkanten ist wenigstens ein Fügemechanismus der vorstehend beschriebenen Art, vorzugsweise in Form eines Rastmechanismus vorgesehen. Alternativ zu dem oder in Kombination mit dem Rastmechanismus ist wenigstens ein band- oder fadenförmiges Fixiermittels vorgesehen, das durch geeignete, innerhalb der Halbschalen angebrachte Befestigungsöffnungen unter Schlaufen- und/oder Knotenbildung fixierbar ist.

Eine weitere Ausführungsform sieht die Ausbildung der Schutzstruktur in Form einer folienartig ausgebildeten Wickelmanschette vor, die jeweils eine einzige, einen zylinderförmigen Hohlraum umfassende, flächige Umwicklung besitzt. Vorzugsweise verfügt die Schutzstruktur in diesem Fall über eine größere Foliendicke verglichen zur Trägersubstratdicke im Bereich des zu einem Tubus gewickelten ersten Trägersubstratbereiches der Cuff-Elektrode, zumal die Schutzstruktur über eine möglichst große materialinhärente Form-erhaltende Materialsteifigkeit verfügen sollte, um eine schützende Haltekraft auf die innenliegende Cuff-Elektrode ausüben zu können. Auch in diesem Fall vermag ein zusätzlicher Fügemechanismus die Schutzstruktur in der geschlossenen Form im Rahmen einer mechanischen Arretierung zu halten.

Neben der reinen Befestigungs- bzw. Haltefunktion sowie des Schutzes vor Gewebewachstum sieht eine weitere bevorzugte Ausführungsform der Schutzstruktur eine elektrosensorische Funktion vor. So verfügt die Schutzstruktur an ihrer im geschlossenen, zweiten Zustand zur Wickelachse radial abgewandten Oberfläche wenigstens eine Kontaktelektrode vor, die mit einer innerhalb der Schutzstruktur integrierten und längs des zweiten Trägersubstratbereiches weiterführenden elektrischen Leiterstruktur verbunden ist. Im implantierten Zustand gelangt auf diese Weise die wenigstens eine an der Außenseite der Schutzstruktur angebrachte Kontaktelektrode in körperlichen und elektrischen Kontakt mit dem extravaskulären Gewebeumfeld, wodurch elektrische Signalabgriffe möglich sind, die in geeigneter Weise zur diagnostischen Signalaufnahme bspw. zum Zwecke eines EKG-Signalabgriffes genutzt werden können.

Die innerhalb der Cuff-Elektrode vorgesehenen Elektroden sowie auch die vorstehend erläuterte, an der äußeren Oberfläche der Schutzstruktur angebrachte wenigstens eine Kontaktelektrode sind jeweils über innerhalb des Trägersubstrates der Cuff-Elektrode bzw. der Schutzstruktur verlaufende elektrische Leitungen kontaktiert, die allesamt zur separat zur Cuff-Elektrode implantierten Versorgungseinheit, vorzugsweise in Form einer Steuer- und elektrischen Energiequelleneinheit, führen.

Die zwischen der Cuff-Elektrode und der implantierbaren elektrischen Versorgungseinheit verlaufende elektrische Zu- und Ableitungsstruktur schließt vorzugsweise über eine elektromechanische Verbindungsstruktur, vorzugsweise in Form einer Steckverbindung an das Trägersubstrat der Cuff-Elektrode an. Die lösungsgemäße Schutzstruktur ist über den wenigstens einen Verbindungsabschnitt entweder unmittelbar mit dem zweiten Trägersubstratbereich der Cuff-Elektrode einstückig verbunden oder mit der elektrischen Zu- und Ableitungsstruktur an oder möglichst nahe an der elektromechanischen Verbindungsstruktur angebracht.

Sowohl das Trägersubstrat der Cuff-Elektrode als auch die folienartige Schutzstruktur sind in Form einer Dünnschicht bzw. eines Dünnfilms ausgebildet und einstückig miteinander verbunden und weisen Dünnschicht- bzw. Dünnfilmdicken im Bereich zwischen 5 µm und 50 µm, vorzugsweise zwischen 5 µm und 20 µm auf.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1a, b: Cuff-Elektrode mit Schutzstruktur in geöffneter Stellung a) sowie in geschlossener Stellung b),
- Fig. 2: Cuff-Elektrode gemäß Stand der Technik,
- Fig. 3a - e: Darstellung unterschiedlicher Belastungszustände der Cuff-Elektrode auf einen Nervenstrang sowie
- Fig. 4a, b: alternative Ausgestaltung der Cuff-Elektrode und Anbringung der Schutzstruktur,
- Fig. 5a, b: alternative Ausgestaltung einer Schutzstruktur in geöffneter Stellung a) sowie in geschlossener Stellung b).
- Fig. 6: Alternative Ausgestaltung und Anbringung der Schutzstruktur.

### Wege zur Ausführung der Erfindung, gewerbliche Verwendbarkeit

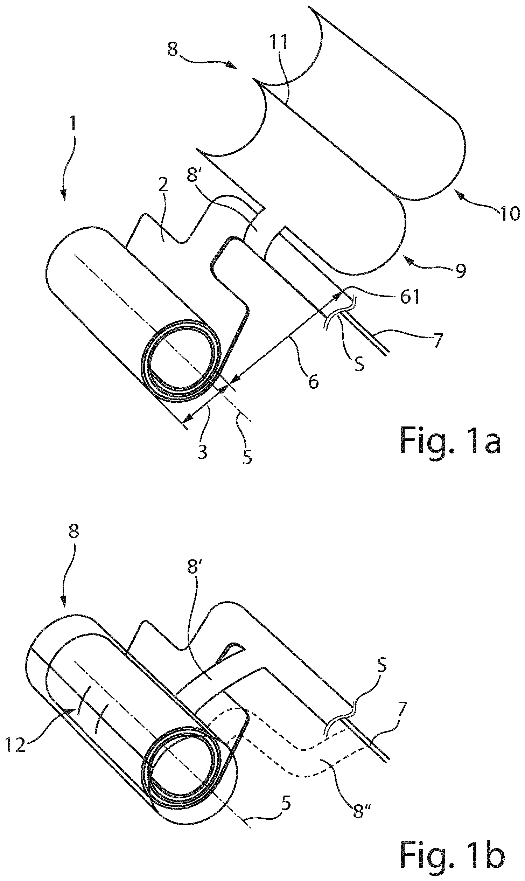

Figur 1a zeigt ein medizinisches Implantat 1 in Form einer Wickelmanschettenelektrode bzw. Cuff-Elektrode gemäß der in Figur 2 bereits erläuterten Darstellung, d.h. der Trägersubstratbereich 3 des Trägersubstrates 2 ist durch wenigstens eineinhalbfaches, vorzugsweise zwei- oder mehrfaches Umwickeln um die Wickelachse geformt und nimmt durch eine Material-inhärent vorgegebene Materialspannung die Form eine Tubus an, in der die Cuff-Elektrode selbstragend bspw. am Umfangsrand eines Nervs ortsfest fixierbar ist. Bereits erläuterte Komponenten des medizinischen Implantates 1 mit bereits eingeführten Bezugszeichen werden zur Vermeidung von Wiederholungen an dieser Stelle nicht erneut beschrieben.

Zum Zwecke eines mechanischen Schutzes des zu einem Tubus um die Wickelachse 5 gewickelten Trägersubstratbereiches 3 der Cuff-Elektrode ist eine Schutzstruktur 8 vorgesehen, die über einen stegartig ausgebildeten Verbindungsabschnitt 8' mit dem Trägersubstrat 2 im Bereich des zweiten Trägersubstratbereiches 6 und insbesondere am stegförmig ausgebildeten Flächenabschnitt 61 verbunden ist. Die Schutzstruktur 8 sowie auch der stegartig ausgebildete Verbindungsabschnitt 8' sind aus dem gleichen folienartigen Material gefertigt, aus dem das Trägersubstrat 2 der Cuff-Elektrode besteht, vorzugsweise aus einer Dünnfilm- bzw. Dünnschicht-Polyimid-Folie, die eine bevorzugte Foliendicke im Bereich zwischen 5 µm und 20 µm aufweist. Der Verbindungsabschnitt 8' und das Trägersubstrat 2 sind vorzugsweise monolithisch verbunden.

Die Schutzstruktur 8 weist zwei zylinderförmige Halbschalen 9, 10 auf, die monolithisch, vorzugsweise über ein Festkörpergelenk 11 miteinander verbunden sind. Das Festkörpergelenk 11 ist vorzugsweise in Form eines Knickfalzes ausgebildet.

Nach Anbringung des medizinischen Implantates 1 längs eines Nervenfaserbündels wird zu Zwecken eines mechanischen Schutzes die in Figur 1a im geöffneten Zustand dargestellte Schutzstruktur 8 in den geschlossenen Zustand gemäß Figur 1b überführt, wodurch beide Halbschalen 9, 10 unter Ausbildung eines geraden Hohlzylinders um den zu einem Tubus gewickelten ersten Trägersubstratbereich 3 zusammengefügt werden. Der in Figur 1b dargestellte geschlossene Zustand der Schutzstruktur 8, bei dem zu einer besseren Illustration beide Halbschalen 9, 10 jeweils transparent dargestellt sind, verdeutlicht, dass die hohlzylinderförmige, geschlossene Form der Schutzstruktur 8 die innenliegende Wickelmanschette sowohl in Umfangsrichtung als auch axial zur Wickelachse 5 vollständig umgibt. Vorzugsweise besitzt die Schutzstruktur 8 im geschlossenen Zustand jeweils beidseitig zur innenliegenden Wickelmanschette ein axiales Übermaß, so dass auf diese Weise verhindert wird, dass die innenliegende Wickelmanschette durch ein Gewebewachstum funktional beeinträchtigt wird.

Die jeweils zu einem vollen geraden Hohlzylinder zusammengefügten Halbschalen 9, 10 der Schutzstruktur 8 können in vorteilhafter Weise mit Hilfe eines lediglich schematisiert dargestellten Fügemechanismus 12 dauerhaft im geschlossenen Zustand gehalten werden.

Alternativ zur Anbringung des stegartig ausgebildeten Verbindungsabschnittes 8' unmittelbar am Flächenabschnitt 61 ist es ebenfalls möglich, die stegartig ausgebildete Verbindungsstruktur 8", siehe gestrichelt angedeutet Verbindungsstruktur 8" in Figur 1b, mit der vom Implantat 1 wegführenden Verbindungsstruktur 8" zu verbinden, vorzugsweise in unmittelbarer Nähe zur Schnittstelle S.

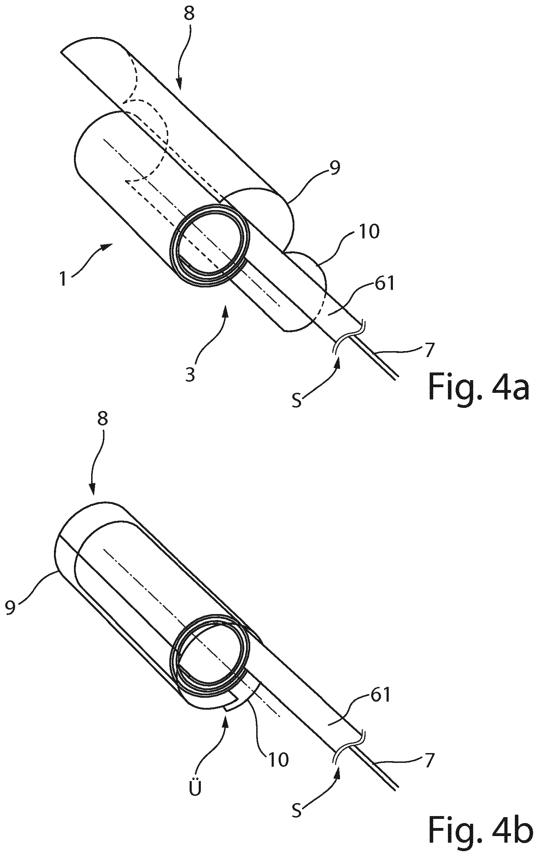

In Figur 4 a ist eine alternative Ausbildung des medizinischen Implantats in Form der Cuff-Elektrode 1 dargestellt, die weitgehend aus einem zu einem Tubus gewickelten Trägersubstratbereich 3 besteht, von dem unmittelbar ein Flächenabschnitt des Trägersubstrats abführt, längs dem die nicht weiter dargestellten elektrischen Leitungen abführen. Die Schutzstruktur 8, die gleichsam der Ausführung in Figur 1a, b aus zwei Halbschalen 9, 10 besteht, schließt unmittelbar an den Trägersubstratbereich 3 an, d.h. ohne jegliche Verbindungsstruktur. In Figur 4b ist der geschlossene Zustand der Schutzstruktur 8 illustriert. Nur aus Gründen besseren Verständnisses ist die Schutzstruktur 8 transparent gargestellt. In dieser Ausführungsform überlappen die Endkanten der Halbschalen 9, 10 längs eines Überlapps Ü.

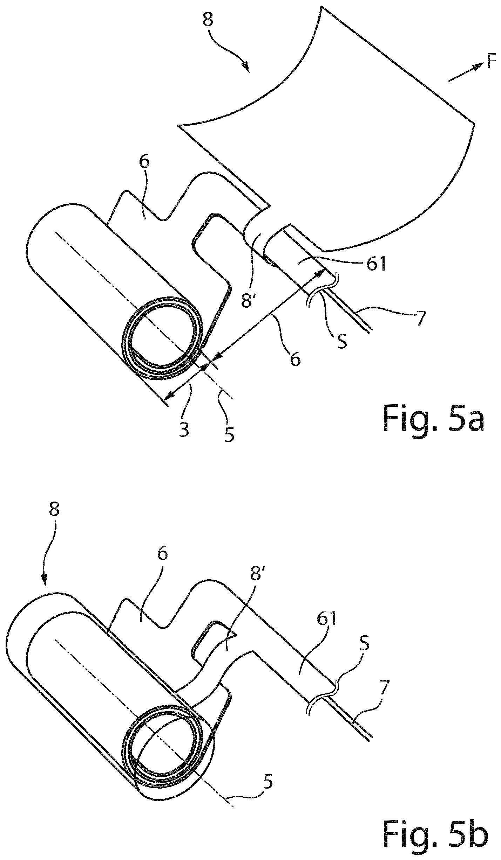

In Figur 5a ist eine Alternative zur Ausbildung der Schutzstruktur 8 illustriert. Die Schutzstruktur 8 ist in diesem Fall in Form eines folienartigen Flächenstückes ausgebildet, in das eine materialinhärente, mechanische Vorspannung derart eingeprägt ist, so dass die Schutzstruktur 8 lediglich unter Aufbringung einer äußeren Kraft F in den in Figur 5a gezeigten offenen Zustand gehalten werden kann. Gleichsam der in Figur 1a illustrierten Schutzstruktur 8 ist auch die flächig ausgebildete Schutzstruktur 8 einstückig mit dem zweiten Trägersubstratbereich 6, insbesondere im Flächenabschnitt 61 über einen stegartig ausgebildeten Verbindungsabschnitt 8' monolithisch verbunden.

Nach Anbringung der Cuff-Elektrode am Nervenstrang wird die Schutzstruktur 8 in den geschlossenen Zustand gemäß Figur 5b überführt, wobei sich der Flächenbereich der Schutzstruktur 8 aufgrund der materialinhärenten mechanischen Vorspannung unter Ausbildung eines geraden Hohlzylinders selbständig umformt. Die Haltekraft, durch die die Schutzstruktur 8 im geschlossenen Zustand gehalten wird, kann vorzugsweise dadurch vergrößert werden, indem die Dicke des Flächenbereiches der Schutzstruktur 8 entsprechend größer gewählt wird, bspw. größer als die Foliendicke im Bereich des Trägersubstrates 2.

Selbstverständlich sind sämtliche bereits erläuterten Maßnahmen hinsichtlich des Vorsehens eines zusätzlichen Fügemechanismus sowie auch einer alternativen Anbringung der Schutzstruktur 8 mit der Verbindungsstruktur 8' sowie auch ohne Verbindungsstruktur, d.h. unmittelbare Anbringung der Schutzstruktur 8 am Trägersubstratbereich 3, vergleichbar der Ausbildung der Cuff-Elektrode gemäß der Figuren 4a, b, auch im Falle des in den Figuren 5a und b dargestellten Ausführungsbeispieles übertragbar möglich.

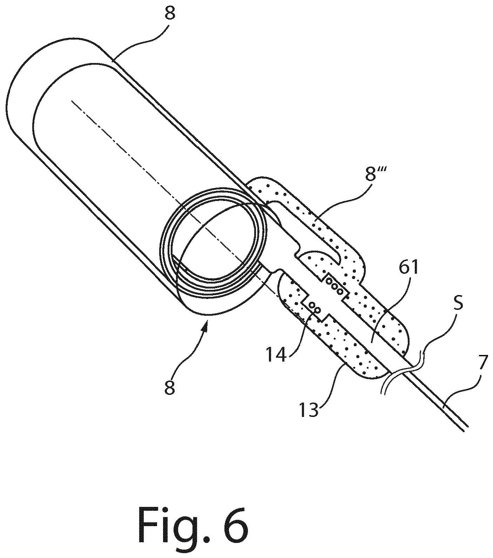

In Figur 6 ist eine Schutzstruktur 8 im geschlossenen Zustand dargestellt, d.h. die Schutzstruktur 8 umfasst die innenliegende Cuff-Elektrode. Schutzstruktur 8 kann in Form zweier Halbschalen, wie in Figuren 1a, b, oder in Art einer Wickelmanschette, wie in Figuren 5 a, b ausgebildet sein. Im Unterschied zu den illustrierten Ausführungsformen ist die Schutzstruktur 8 in diesem Fall über eine Verbindungsstruktur 8‴ mit einer aus elastomeren Material, vorzugsweise aus Silikon oder aus einem auf Silikon basierenden Kunststoff gefertigten Ummantelung 13 verbunden, die zumindest einen Abschnitt des Flächenabschnittes 61 umgibt. Die vorzugsweise schlauchförmige Ummantelung 8 ist an den folienartigen Flächenabschnitt 61 über eine einstückig am Flächenabschnitt 61 angebrachte Verankerungsstruktur 14 im Wege einer Formschlussverbindung gefügt. Der Flächenabschnitt 61 ist Teil des Trägersubstrates 2 und schließt mittel- oder unmittelbar an den zu einem Tubus gewickelten Trägersubstratbereich 3 der Cuff-Elektrode an. Form und Größe der Ummantelung 13 sind derart gewählt, dass die Cuff-Elektrode zum Zwecke der intrakorporalen Anbringung an einem Nervenfaserbündel für einen Arzt einfacher handhabbar ist. Nicht notwendigerweise, jedoch in vorteilhafter Weise ist die Schutzstruktur 8 in diesem Fall aus dem gleichen Material gefertigt, aus dem auch die Ummantelung 13 besteht.

### Bezugszeichenliste

- 1: medizinisches Implantat
- 2: Trägersubstrat
- 3: erster Trägersubstratbereich
- 4: freies Abschnittsende
- 5: Wickelachse
- 6: zweiter Trägersubstratbereich
- 61: stegartiger Flächenabschnitt
- 7: Verbindungsstruktur
- 8: Schutzstruktur
- 8', 8", 8‴: Verbindungsabschnitt
- 9, 10: Halbschalen
- 11: Festkörpergelenkt
- 12: Fügemechanismus
- 13: Ummantelung
- 14: Verankerungsstruktur
- Ü: Überlapp

## Patentansprüche

1. Medizinisches Implantat in Art einer Wickelmanschetten-Elektrodenanordnung, kurz Cuff-Elektrode, die ein flexibles, biokompatibles, folienartiges Trägersubstrat aufweist, das einen Trägersubstratbereich aufweist, der durch Wickeln um eine Wickelachse die Gestalt eines Tubus annimmt, **dadurch gekennzeichnet, dass** eine Schutzstruktur unmittelbar oder mittelbar an dem Trägersubstrat angebracht ist, die aus einem ersten, geöffneten Zustand in einen zweiten, geschlossenen und den zu einem Tubus gewickelten Trägersubstratbereich zumindest axial zur Wickelachse bereichsweise und in Umfangsrichtung des Tubus vollständig umfassenden Zustand überführbar ist.

2. Medizinisches Implantat nach Anspruch 1,
wobei die Schutzstruktur folienartig ausgebildet ist.

3. Medizinisches Implantat nach Anspruch 1 oder 2,
wobei die Schutzstruktur im geschlossenen zweiten Zustand den zu einem Tubus gewickelten Trägersubstratbereich (3) axial und in Umfangsrichtung vollständig überdeckt.

4. Medizinisches Implantat nach einem der Ansprüche 1 bis 3,
wobei an der Schutzstruktur ein Fügemechanismus angebracht ist, der unter Ausbildung einer Form-und/oder Kraftschlussverbindung die Schutzstruktur im zweiten, geschlossenen Zustand lösbar fest arretiert.

5. Medizinisches Implantat nach einem der Ansprüche 1 bis 4,
wobei die Schutzstruktur über wenigstens einen stegartig ausgebildeten Verbindungsabschnitt mittel- oder unmittelbar mit dem Trägersubstrat verbundenen ist.

6. Medizinisches Implantat nach einem der Ansprüche 1 bis 5,
wobei die Schutzstruktur in Form zweier zylinderförmiger Halbschalen ausgebildet ist, die in gegenseitiger Ergänzung einen geradzylinderförmigen Hohlraum einschließen.

7. Medizinisches Implantat nach Anspruch 6,
wobei die zwei zylinderförmigen Halbschalen über ein Festkörpergelenk einstückig miteinander verbunden sind und aus dem geöffneten Zustand in einen den zylinderförmigen Hohlraum umfassenden, geschlossenen Zustand überführbar sind.

8. Medizinisches Implantat nach einem der Ansprüche 1 bis 5,
wobei die Schutzstruktur in Form einer folienartig ausgebildeten Wickelmanschette ausgebildet ist, die eine, einen zylinderförmigen Hohlraum umfassende, flächige Umwicklung aufweist.

9. Medizinisches Implantat nach einem der Ansprüche 1 bis 8,
wobei die Schutzstruktur im geschlossenen zweiten Zustand eine zur Wickelachse radial abgewandte Oberfläche besitzt, an der wenigstens eine Kontaktelektrode angebracht ist, die mit einer innerhalb der Schutzstruktur integrierten und längs des Trägersubstrats weiterführenden elektrischen Leiterstruktur verbunden ist.

10. Medizinisches Implantat nach einem der Ansprüche 1 bis 9,
wobei mittel- oder unmittelbar an den zu einem Tubus gewickelten Trägersubstratbereich ein zweiter Trägersubstratbereich einstückig anschließt, längs dem eine elektromechanische Verbindungsstruktur angebracht ist, an die eine zu einer implantierbaren elektrischen Versorgungseinheit führende, elektrische Zu- und Ableitungsstruktur gefügt ist, und
dass die Schutzstruktur mittel- oder unmittelbar an der elektrischen Zu- und Ableitungsstruktur angebracht ist.

11. Medizinisches Implantat nach Anspruch 10,
wobei zumindest abschnittweise der zweite Trägersubstratbereich mit einer Ummantelung aus einem elastischen Material umgeben ist.

12. Medizinisches Implantat nach Anspruch 11,
wobei die Schutzstruktur einstückig mit der Ummantelung verbunden ist.

13. Medizinisches Implantat nach einem der Ansprüche 1 bis 10,
wobei die Schutzstruktur einstückig mit dem Trägersubstrat verbunden ist.

14. Medizinisches Implantat nach einem der Ansprüche 1 bis 13,
wobei das Trägersubstrat in dem ersten Trägersubstratbereich eine Materialvorspannung eingeprägte ist, durch die das Trägersubstrat ohne äußere Krafteinwirkung eine vordefinierte Wickelkonfiguration einnimmt, bei der ein freies Abschnittsende des Trägersubstratbereiches von diesem wenigstens einlagig lose, d.h. von wenigstens einer Substratwicklung radial überdeckt ist.

15. Medizinisches Implantat nach einem der Ansprüche 2 bis 14,
wobei das folienartige Trägersubstrat und die folienartige Schutzstruktur jeweils als Dünnschicht oder Dünnfilm ausgebildet sind und eine Schicht- bzw. Filmdicke von 5 µm bis 50 µm, vorzugsweise von 5 µm bis 20 µm aufweisen.

16. Medizinisches Implantat nach einem der Ansprüche 1 bis 15,
wobei der aus dem flexiblen, biokompatiblen, folienartigen Trägersubstrat bestehende Trägersubstratbereich durch wenigstens eineinhalbfaches, vorzugsweise wenigstens zweifaches Wickeln um die Wickelachse die Gestalt eines Tubus annimmt.

## Claims

1. A medical implant in the form of a wrap-around cuff electrode assembly, cuff electrode in short, comprising a flexible, biocompatible, film-like carrier substrate, that has a carrier substrate region which, through wrapping around a wrap axis, assumes the form of a tube **characterised in that** a protective structure is applied directly or indirectly to the carrier substrate, that can be transferred from a first, open state into a second, closed state enclosing the carrier substrate region at least axially to the wrap axis in parts and completely in the peripheral direction of the tube.

2. The medical implant according to claim 1,
wherein the protective structure is film-like in design.

3. The medical implant according to claim 1 or 2,
wherein in that the protective structure in the closed, second state completely covers the carrier substrate region (3) of the cuff electrode wound to form a tube axially and completely in the peripheral direction.

4. The medical implant according to any of claims 1 to 3,
wherein a joining mechanism is applied on the protective structure which, forming a positive and/or non-positive connection, firmly secures the protective structure in the second, closed state in a releasable manner.

5. The medical implant according to any one of claims 1 to 4,
wherein the protective structure is directly or indirectly connected via at least one web-like connecting section to the carrier substrate.

6. The medical implant according to any one of claims 1 to 5,
wherein the protective structure is in the form of two cylindrical half shells which, mutually complementing each other, enclose a straight cylindrical hollow space.

7. The medical implant according to claim 6,
wherein the two cylindrical half shells are connected to each other by means of a solid body joint and can be transferred from the open state into a closed state enclosing the cylindrical hollow space.

8. The medical implant according to any one of claims 1 to 5,
wherein the protective structure is in the form of a film-like wrap-around cuff, which comprises a planar wrapping enclosing a cylindrical hollow space.

9. The medical implant according to any of claims 1 to 8,
wherein the protective structure in the closed, second state has a surface radially facing away from the wrap axis on which at least one contact electrode is applied which is connected with an electrical conducting structure integrated within the protective structure and extending along the carrier substrate.

10. The medical implant according to any one of claims 1 to 9,
wherein directly or indirectly adjoining in one piece the carrier substrate region wrapped to form a tube, is a second carrier substrate region along which an electromechanical connecting structure is applied, to which an electrical supply and discharge structure is joined that leads to an implantable electrical supply unit, and
in that the protective structure is directly or indirectly attached to the electrical supply and discharge structure.

11. The medical implant according to claim 10,
wherein, at least in sections, the second carrier substrate region is surrounded by a sheathing made of an elastic material.

12. The medical implant according to claim 11,
wherein the protective structure is connected in one piece to the sheathing.

13. The medical implant according to any one of claims 1 to 10,
wherein the protective structure is connected in one piece to the carrier substrate.

14. The medical implant according to any one of claims 1 to 13,
wherein in the first carrier substrate region, a material pre-tensioning is introduced into the carrier substrate through which, without the effect of external force, the carrier substrate assumes a predefined wrapping configuration in which a free section end of the carrier substrate region is radially covered thereby loosely in at least one layer, i.e. by at least one substrate wrapping.

15. The medical implant according to any one of claims 2 to 14,
wherein the film-like carrier substrate and the film-like protective structure are each designed in the form of a thin layer or thin film, and have a layer or film thickness of 5 µm to 50 µm, preferably of 5 µm to 20 µm.

16. The medical implant according to any one of claims 1 to 15,
wherein the carrier substrate region consisting of the flexible, biocompatible, film-like carrier substrate, assumes the shape of a tube by wrapping one and half times, preferably at least two times around the wrap axis.

## Revendications

1. Implant médical sous forme d'un agencement d'électrodes à manchon d'enroulement, en bref électrode à manchon, qui comporte un substrat porteur souple, biocompatible, de type à film, qui comporte une zone de substrat porteur, qui adopte la structure d'un tube par enroulement autour d'un axe d'enroulement,
**caractérisé en ce qu'**une structure de protection est placée directement ou indirectement sur le substrat porteur, qui peut être transférée d'un premier état ouvert dans un deuxième état fermé comprenant par endroits la zone de substrat porteur enroulée à un tube au moins axialement par rapport à l'axe d'enroulement et complètement en direction périphérique du tube.

2. Implant médical selon la revendication 1, sachant que la structure de protection est constituée en forme de film.

3. Implant médical selon la revendication 1 ou 2, sachant que la structure de protection recouvre complètement au deuxième état fermé axialement et en direction périphérique la zone de substrat porteur (3) enroulée à un tube.

4. Implant médical selon l'une quelconque des revendications 1 à 3, sachant qu'un mécanisme de jonction est placé sur la structure de protection, qui bloque fermement de façon amovible la structure de protection dans le deuxième état fermé en formant une liaison par conformité de forme et/ou de force.

5. Implant médical selon l'une quelconque des revendications 1 à 4, sachant que la structure de protection est reliée indirectement ou directement au substrat porteur par au moins une section de liaison constituée comme une nervure.

6. Implant médical selon l'une quelconque des revendications 1 à 5, sachant que la structure de protection est constituée sous la forme de deux demi-coques cylindriques, qui forment en complémentarité réciproque un espace creux cylindrique droit.

7. Implant médical selon la revendication 6, sachant que les deux demi-coques cylindriques sont reliées en une pièce l'une à l'autre par une articulation à corps solide et peuvent être transférées de l'état ouvert à un état fermé comprenant un espace creux cylindrique.

8. Implant médical selon l'une quelconque des revendications 1 à 5, sachant que la structure de protection est constituée sous la forme d'un manchon d'enroulement constitué en forme de film, qui comporte un enroulement plane comprenant un espace creux cylindrique.

9. Implant médical selon l'une quelconque des revendications 1 à 8, sachant que la structure de protection possède au deuxième état fermé une surface radialement opposée à l'axe d'enroulement sur laquelle est placée au moins une électrode de contact, qui est reliée à une structure conductrice électrique intégrée à l'intérieur de la structure de protection et continuant le long du substrat porteur.

10. Implant médical selon l'une quelconque des revendications 1 à 9, sachant qu'une deuxième zone de substrat porteur se raccorde en une seule partie indirectement ou directement à la zone de substrat porteur enroulée à un tube, est placée le long de la structure de liaison électromécanique, est jointe à la structure d'arrivée et de dérivation électrique menant à une unité d'alimentation électrique implantable et sachant que la structure de protection est placée indirectement ou directement sur la structure d'arrivée et de dérivation électrique.

11. Implant médical selon la revendication 10, sachant qu'au moins par tronçons, la deuxième zone de substrat porteur est entourée d'une gaine faite d'un matériau élastique.

12. Implant médical selon la revendication 11, sachant que la structure de protection est reliée en une pièce à la gaine.

13. Implant médical selon l'une quelconque des revendications 1 à 10, sachant que la structure de protection est reliée en une pièce au substrat porteur.

14. Implant médical selon l'une quelconque des revendications 1 à 13, sachant qu'une précontrainte matérielle est empreinte au substrat porteur dans la première zone de substrat porteur par laquelle le substrat porteur adopte sans effet de force extérieur une configuration d'enroulement prédéfinie pour laquelle une extrémité de section libre de la zone de substrat porteur est radialement couverte par celle-ci au moins en une couche de façon détendue, c'est-à-dire par au moins un enroulement de substrat.

15. Implant médical selon l'une quelconque des revendications 2 à 14, sachant que le substrat porteur en forme de film et la structure de protection en forme de film sont constitués respectivement sous la forme d'une couche mince ou d'un film mince et comportent une épaisseur de couche ou de film de 5 µm à 50 µm, de préférence de 5 µm à 20 µm.

16. Implant médical selon l'une quelconque des revendications 1 à 15, sachant que la zone de substrat porteur composée du substrat porteur souple, biocompatible, en forme de film adopte la structure d'un tube par au moins un enroulement et demi, de préférence au moins deux enroulements autour de l'axe d'enroulement.
